# EUROPEAN PATENT SPECIFICATION

(11) **EP 1 508 616 B1**
(45) Date of publication and mention of the grant of the patent: **25.10.2006**
(21) Application number: 04077437.4
(22) Date of filing: 23.04.1999
(51) Int. Cl.: C12N 9/96, C12N 9/10

(54) **Method for stabilizing an aspartate aminotransferase and the corresponding compositions**
Methode zum Stabilisieren von Aspartataminotransferase und die entsprechenden Zusammensetzungen
Méthode de stabilisation d'aspartate aminotransferase et compositions correspondantes

(30) Priority: 24.04.1998 JP 13115998
(43) Date of publication of application: 23.02.2005
(62) Divisional of application: 99917154.9
(73) Proprietor: SYSMEX CORPORATION, Chuo-ku Kobe-shi Hyogo 651-0073 (JP)
(72) Inventor: Tabata, Hiromasa, c/o International Reagents Corp., Kobe.shi, Hyogo 651-2241 (JP); Baba, Toshiyuki, c/o International Reagents Corp., Kobe-shi, Hyogo 651-2241 (JP); Nagamatsu, K., c/o International Reagents Corp., Kobe-shi, Hyogo 651-2241 (JP); Watazu, Y., c/o International Reagents Corp., Kobe-shi, Hyogo 651-2241 (JP); Aoki, Ryoji, c/o Asahi Chemical Industry Co. Ltd., Tagata-gun, Shizuoka 410-2321 (JP)
(74) Representative: Williams, Paul Edwin

(56) References cited:
- EP-A- 0 791 658
- GB-A- 866 423
- SEGAL H L ET AL: "INTRACTION OF RAT LIVER ENZ ALANINE AMINO TRANSFERASE WITH L PROLINE" BIOCHEMICAL AND BIOPHYSICAL RESEARCH COMMUNICATIONS, vol. 30, no. 1, 1968, pages 63-68, XP001063379 ISSN: 0006-291X
- JACOBI R AND GÖCKERITZ D: "Stabilität und Stabilisierung von Enzymen" PHARMAZIE, VEB VERLAG VOLK UND GESUNDHEIT. BERLIN, DD, vol. 44, no. 10, October 1989 (1989-10), pages 678-685, XP002102673 ISSN: 0031-7144

## Description

### Technical Field

The present invention relates to a method of stabilizing an enzyme contained in a medium and an enzyme composition, with regard to a control substance. The control substance is used to fulfill the following roles, that is, those containing a certain amount of an enzyme as a component are used as the control substance and applied to an analyzer in an test for measuring the amount of the enzyme contained in human blood, whereby the analyzer inspects whether or not an accurate value of the enzyme can be detected. Alternatively, the numerical value of measurement of the control substance is previously obtained, whereby an accurate amount of the enzyme is obtained from the measurement value obtained in an actual specimen by proportioning.

### Background Art

Aspartate aminotoransferase EC 2.6.1.1 (hereinafter abbreviated to AST) is an enzyme capable of producing glutamic acid and oxaloacetic acid from aspartic acid and α-ketoglutaric acid and mainly exists in heart, liver and skeletal muscle, and is a useful index for diagnosis of acute hepatitis, chronic hepatitis and cardiac infarction.

In an ordinary test performed to measure the amount of AST contained in blood, the control substance is often used.

Accordingly, handling of the control substance has become important to secure the stability and reliability of an test value in an ordinary test and to perform a clinical trial to which an accurate and high-level technique is required. For example, in case the measurement value of AST contained in human blood is detected, a control substance containing AST is used. However, it is necessary to sufficiently stabilize the enzyme activity of AST as an unstable enzyme incorporated in the control substance to sufficiently fulfill the roles of the control substance described above.

From such a point of view, the prior arts with the object of stabilizing the enzyme incorporated in the control substance are disclosed in Unexamined Patent Publication (Kokai) No. Sho 55-141194, Unexamined Patent Publication (Kokai) No. Sho 56-148291 and Unexamined Patent Publication (Kokai) No. Sho 57-45453. In these prior arts, ethylene glycol, sucrose or glycerol is used as the stabilizer.

The stabilization of the enzyme using amino acid has hitherto been studied by Harold L. Segal et. al. (Biochemical and Biophysical Research Community cations, Vol. 30 (1), pages 63-68, 1968).

Although there is also a report using amino acid to reduce the turbidity of the control substances, it is considered that amino acid can prevent denaturation of protein in either case.

However, a conventional stabilizer such as ethylene glycol, sucrose or glycerol must be used in high concentration so that the stabilizer exhibits the effect thereof. That is, the concentration of ethylene glycol and that of glycerin must be became higher, for example, about 5 mol/L and 3.3 mol/L, while sucrose must be added in the proportion within a range from 1 to 10%. Therefore, the control substance itself has high specific gravity and/or high viscosity to cause such a problem that the control substance, which should serve as a parameter, has physical properties different from those of human serum. Such a problem leads to the state where an intrinsic object of the control substance itself can not be attained, for example, it produces a factor for causing a difference in measured value between types of a latest automatic analyzer and equipments when applying the control substance to the analyzer because the accuracy of sampling is different from that of common human serum (Japan Clinical Chemistry Society, Scientific Liaison Committee, Clinical Chemistry Vol. 25 (2), pages 135-148, 1996).

### Disclosure of the Invention

To solve the problems described above, the present inventors have studied intensively based on the fact such as roles of amino acid to be fulfilled to the turbidity of the control substances and denaturation of protein. As a result, they have found that - proline particularly stabilizes the enzyme AST contained in the control substance, thus completing the method of stabilizing an enzyme and enzyme composition of the present invention.

The present invention provides a method for stabilizing aspartate aminotransferase in at least one medium selected from the group consisting of serum and buffer, the method comprising adding proline to said medium.

The proline concentration is preferably within a range from 0.5 to 500 mmol/L.

The serum or buffer is preferably a buffer containing a soluble protein.

The soluble protein is preferably at least one soluble protein selected from the group consisting of albumin and gelatin.

Preferably, the soluble protein is albumin in a concentration within a range from 0.5 to 15W/V%, or gelatine in a concentration within a range from 0.5 to 15W/V%.

The present invention also provides an enzyme composition comprising at least one medium selected from the group consisting of serum and buffer, aspartate aminotransferase, and proline.

In the above description, the medium refers to a mother liquor such as solvent or dispersion medium in which the enzyme and stabilizer are dissolved or dispersed, and those prepared by dissolving or dispersing the enzyme and stabilizer in the medium can be used as the control substance. In case the object to be test is contained in human serum, human serum or a similar one prepared by treating the human serum is preferably used as the medium for control substance. That is, it is preferable to select, as the medium, those having properties similar to those of the object to be examined.

As the medium, for example, serum and buffer can be used. The serum refers to human serum, serum of other animals, or treated serums in a broad sense. The serum and buffer may be a buffer containing a soluble protein solution. Hereinafter, aspartate aminotransferase is abbreviated to AST, while alanine aminotransferase is abbreviated to as ALT.

Proline as the stabilizer for stabilizing AST is used alone.

In the features described above, those containing AST as the enzyme in the medium can be used as the control substance, typically, in case the amount of AST contained in human blood is measured.

The origin of AST to be incorporated in the control substance includes, but is not specifically limited to, biological substance such as bovine heart, pig heart, human heart, serum, red blood cell and urine, those prepared by cultivating human cells or those prepared by cultivating a transforming cell integrated with a human-derived gene. In this case, the content of AST may be controlled within a range from 5 to 1000 U/L, and preferably from 30 to 500 U/L.

In the features described above, when the medium is a buffer, for example, a BES buffer prepared by dissolving bovine serum albumin can be used. However, those prepared by dissolving various substances in buffet can be used to have chemically or physically similar properties corresponding to types and states of the object to be examined (strictly a medium of the object to be examined) to be subjected actually to an examining apparatus. The present invention also includes such a case.

The buffer used in the present invention includes, for example, organic amine and Good's buffers whose pH can be adjusted appropriately within a range from 6.0 to 8.5; and biochemical buffers such as citric acid-sodium diphosphate buffer, hydrochloric acid-sodium veronal-sodium acetate buffer, potassium monophosphate-sodium diphosphate buffer, potassium monophosphate-borate buffer, potassium monophosphate-sodium hydroxide buffer, hydrochloric acid-collidine buffer, hydrochloric acid-sodium veronal buffer, hydrochloric acid-tris(hydroxymethyl)aminomethane buffer, hydrochloric acid-borate buffer, boric acid-sodium carbonate buffer, boric acid-borate buffer, hydrochloric acid-aminomethylpropanediol buffer, ammonium chloride-ammonia buffer, glycine-sodium hydroxide buffer, boric acid-sodium hydroxide buffer, hydrochloric acid-sodium dimethylglycine buffer, borate-sodium hydroxide buffer, borate-sodium carbonate buffer, Sörensen buffer, glycine-sodium chloride-hydrochloric acid buffer, sodium dicitrate-hydrochloric acid buffer, sodium diacetate-sodium hydroxide buffer, borate-sodium hydrochloride buffer, Michaerlis buffer, sodium veronal-sodium acetate-hydrochloric acid buffer, Clark-Lub's buffer, boric acid-potassium hydrochloride-sodium hydroxide buffer, Atkins-Pantin buffer, Palitzsch buffer, Kolthoff buffer, McIlvaine buffer, Hasting-Sendroy buffer, Britton-Robinson buffer, maleate buffer, tris-maleate buffer, veronal buffer and veronal-acetate buffer. Buffers other than these buffers can also be used as far as they have buffering capacity at the pH within a range from 6.0 to 8.5.

The organic amine buffer includes, for example, diethanolamine buffer, 2-ethylaminoethanol buffer, 2-amino-2-methyl-1-propanol and N-methyl-D-glucamine.

The Good's buffer includes, for example, MES (2-(N-Morphilino)ethanesulfonic acid) buffer, Bis-Tris (Bis(2-hydroxyethyl)iminotris(hydroxymethyl)methane) buffer, ADA (N-(2-Acetamido)iminodiacetic acid) buffer, PIPES (Piperazine-N,N'-bis(2-ethanesulfonic acid) buffer, ACES (N-(2-Acetamido)-2-aminoethanesulfonic acid) buffer, MOPSO (3-(N-Morpholino)-2-hydroxypropanesulfonic acid) buffer, BES (N,N-Bis(2-hydroxyethyl)-2-aminoethanesulfonic acid) buffer, MOPS (3-(N-Morpholino)propanesulfonic acid) buffer, TES (N-Tris(hydroxymethyl)methyl-2-aminoethanesulfonic acid) buffer, HEPES (N-2-hydroxyethylpiperazine-N'-2-ethanesulfonic acid) buffer, DIPSO (3-[N,N-Bis(2-hydroxyethyl)amino]-2-hydroxypropanesulfonic acid) buffer, TAPSO (N-Tris(hydroxymethyl)methyl-2-hydroxy-3-aminopropanesulfonic acid) buffer, POPSO (Piperazine-N,N'-bis(2-hydroxypropanesulfonic acid) buffer, HEPPSO (N-2-hydroxyethylpiprrazine-N-2-hydroxypropane-3-sulfonic acid) buffer, EPPS (N-2-Hydroxyethylpiperazine-N'-3-propanesulfonic acid, another name: HEPPS) buffer, Tricine (Tris(hydroxymethyl)methylglycine) buffer, Bicine (N,N-bis(2-hydroxyethyl)glycine) buffer, TAPS (N-tris(hydroxymethyl)methyl-3-aminopropanesulfonic acid) buffer, CHES (2-(Cyclohexylamino)ethanesulfonic acid) buffer, CAPSO (3-N-Cyclohexylamino-2-ydroxypropanesulfonic acid) buffer and CAPS (3-Cyclohexylaminopropanesulfonic acid) buffer.

When using the organic amine buffer, it may be used as an aqueous medium whose concentration was adjusted within a range from 20 mM to 2 M, preferably from 20 mM to 1 M, and most preferably from 20 mM to 500 mM. Preferable aqueous medium is specifically purified water, and coenzymes, soluble salts, surfactants, stabilizers and antiseptics may also be appropriately incorporated.

When using the Good's buffer or biochemical buffer, it may be used after adjusting the concentration within a range from 20 mM to 1 M, preferably from 20 mM to 500 mM, and most preferably from 20 mM to 300 mM.

In the features described above, when the medium is a soluble protein solution, the soluble protein solution includes aqueous solutions of BSA, human serum albumin (HSA) and gelatin and these aqueous solutions can be used alone or in combination. In this case, the concentration of albumin and that of gelatin are respectively within a range from 0.5 to 15W/V%. When the medium is serum, the buffers described above can also be appropriately used.

Proline is not added in a large amount so that the resulting control substance has neither high specific gravity, nor high viscosity.

The content of proline is preferably controlled within a range from 0. 5 to 500 mmol/L. More preferably, the content of proline is controlled within a range from 100 to 500 mmol/L.

It is made possible to exert good stabilization effect and to enable physical and chemical properties to bear resemblance to those of serum as the object to be examined by controlling each content within the above range. Thus, it is made possible to continually make physical and chemical properties of the control substance resemble to those of serum as the object to be examined, secure the stability, obtain the reliability of the resulting data, and eliminates a difference between equipments where the examination is performed.

According to the method of stabilizing AST of the present invention, it is made possible to inhibit denaturation and thereby stabilize AST in a medium by incorporating proline.

It is also made possible to stabilize AST by the stabilizer and to obtain stable and accurate detected data in the examination whose object to be examined is AST . By using proline as the stabilizer to AST, particularly, this enzyme can be sufficiently stabilized by a small content of proline.

According to the method of stabilizing AST of the present invention, it is made possible to use the control substance wherein AST as well as proline as the stabilizer thereof are added in serum as the medium and to detect under good environment which is physically and chemically similar when AST contained in serum is detected by using the medium as serum.

Similarly, AST can be stabilized with proline as the stabilizer in a stable medium such as buffer by using the medium as the buffer. As a matter of course, a control substance having physical and chemical properties similar to those of the object to be examined (solvent) by optionally dissolving various components in the buffer.

In the method of stabilizing AST of the present invention, when using proline, AST can be satisfactorily stabilized by controlling the proline content within a range from 0.5 to 500 mmol/L.

In the method of stabilizing AST of the present invention, in case the serum or buffer is buffer containing a soluble protein, AST as the stabilizer can be satisfactorily stabilized by proline.

In case the soluble protein is at least one soluble protein selected from the group consisting of albumin and gelatin, AST can be stabilized by proline as the stabilizer in a buffer containing albumin or gelatin. In this case, AST can be preferably stabilized when the concentration of albumin and that of gelatin are respectively within a range from 0.5 to 15W/V%.

According to the enzyme composition of the present invention, there can be provided an enzyme composition, which is not liable to cause denaturation of AST and can exist in the stable state, by incorporating; AST and proline in at least one medium selected from the group consisting of serum and buffer. Therefore, it is made possible to obtain stable and accurate detected data by using such an enzyme composition, as the control substance, in the examination whose object to be examined is AST.

### Best Mode for Carrying Out the Invention

Examples of the preparation of a control substance comprising proline as a stabilizer and AST as an enzyme component according to the present invention will now be described.

Using human serum (manufactured by TORINA CO. (Switzerland)), an endogenous enzyme is devitalized by previously subjecting to a heat treatment at 56°C for four hours and then sterilized by filtration using a membrane filter of 0.2 µm in pore size (solution thus obtained is referred to as a human serum base).

For example, 300 mmol/L of proline is dissolved in the human serum base and a certain amount of AST is dissolved furthermore, thereby making it possible to obtain a control substance containing proline as a stabilizer and AST

Similarly, examples of the preparation of a control substance comprising proline as a stabilizer, AST as an enzyme component and a buffer as a medium according to the present invention will now be described.

Bovine serum albumin of 3W/V% (manufactured by INTERGEN CO. (U.S.A)) is incorporated in a BES buffer (20 mmol/L) and then sterilized by filtration (pH 7.4, solution thus obtained is referred to as a BSA base).

For example, 300 mmol/L of proline is dissolved in the BSA base and a certain amount of AST is dissolved furthermore, thereby making it possible to obtain a control substance containing proline as a stabilizer and AST in the buffer medium.

Furthermore, the control substance obtained by the method of the present invention is in the state of liquid on common use, but may be in the state other than liquid, such as freeze-dried product, cold-stored product and frozen liquid product.

### Examples

The following examples further illustrate the present invention, but the present Invention Is not limited by the

### examples.

### Example 1

Confirmation of stabilization effect of various amino acids on AST in control substance

Using a human serum base and a BSA base as a medium, control substances were prepared respectively by adding, as a stabilizer, no amino acid, valine (10 mmol/L) as amino acid, proline (10 mmol/L) or other amino acid (10 mmol/L, provided that 1 mmol/L in case of tyrosine) to the respective mediums and then adding, as an enzyme, AST (about 100 U/L)

The respective control substances thus obtained were stored at 45°C for four days and the residual activity of AST contained in the respective control substances was measured.

AST used herein is AST derived from a human liver gene recombinant (hereinafter referred to as r-AST) manufactured by ASAHI CHEMICAL INDUSTRIES CO., LTD. (manufacturing No. T-70).

Furthermore, the human serum base used herein is a human serum base prepared by previously subjecting human serum to a heat treatment at 56°C for four hours thereby to devitalize an endogenous enzyme, followed by sterilization by filtration using a membrane filter of 0.2 µm in a pore diameter. The BSA base used herein is BES buffer (20 mmol/L) containing 3W/V% BSA.

With regard to the residual activity of AST, the residual activity of AST was measured by using an AST reagent L "KOKUSAI" manufactured by INTERNATIONAL REAGENTS CORPORATION.

The results are shown in Table 1.

**Table 1**

| Stabilizer | Medium | | | |
|---|---|---|---|---|
| Amino acid (10 mmol/L) | BSA base | | Human serum base | |
| | Residual activity (%) of AST | | Residual activity (%) of AST | |
| No addition | 53 | | 41 | |
| Valine | 85 | | 71 | |
| Proline | 73 | | 54 | |
| Alanine | 39 | | 33 | |
| Leucine | 46 | | 40 | |
| Isoleucine | 50 | | 42 | |
| Methionine | 48 | | 40 | |
| Tryptophane | 52 | | 38 | |
| Phenylalanine | 53 | | 40 | |
| Glycine | 53 | | 40 | |
| Serine | 52 | | 40 | |
| Threonine | 50 | | 37 | |
| Cysteine | 47: | | 32 | |
| Tyrosine | 49 | | 40 | |
| Asparagine | 41 | | 37 | |
| Glutamine | 43 | | 32 | |
| Lysine | 53 | | 39 | |
| Histidine | 47 | | 36 | |
| Arginine | 50 | | 39 | |
| Aspartic acid. | 32 | | 20 | |
| Glutamic acid | 31 | | 25 | |

As is apparent from Table 1, proline exerted preferable stabilization effect on AST

### Example 2

Confirmation of stabilization effect of proline on AST in control substance

Using a human serum base and a BSA base as medium, control substances were prepared respectively by adding, as a stabilizer, no proline and proline in the amount of 0, 5. 10, 100, 300 or 500 mmol/L to the respective mediums and then adding, as an enzyme, r-AST (about 100 U/L)

The respective control substances thus obtained were stored at 45°C for four days and the residual activity of AST contained in the respective control substances was measured. The results are shown in Table 2

**Table 2**

| Stabilizer | Medium | | | |
|---|---|---|---|---|
| Concentration of proline (mmol/L) | BSA base | | Human serum base | |
| | Residual activity (%) of AST | | Residual activity (%) of AST | |
| 0 | 53 | | 41 | |
| 0.5 | 60 | | 49 | |
| 10 | 65 | | 54 | |
| 100 | 74 | | 67 | |
| 300 | 84 | | 82 | |
| 500 | 84 | | 85 | |

As is apparent from Table 2, the residual activity of AST was 53% in case of no addition of proline in the BSA base, while the residual activity of AST became 60% by adding 0. 5 mmol/L of proline, thus improving the stability. The residual activity of AST became 74% by adding 100 mmol/L of proline.

Furthermore, the residual activity of AST was 84% when the concentration of proline is 300 mmol/L, while tho residual activity of AST was 84% when the concentration of proline is 500 mmol/L.

Similarly, the residual activity of AST was 41% in case of no addition of proline in the human serum base, while the residual activity of AST became 49% by adding 0.5 mmol/L of proline, thus improving the stability. The residual activity of AST became 67% by adding 100 mmol/L of proline, while the residual activity of AST became 82% by adding 300 mmol/L of proline. Furthermore, the residual activity of AST was 85% when the concentration of proline is 500 mmol/L.

As is apparent from the above description, the concentration of proline is preferably controlled within a range from 0.5 to 500 mmol/L, and more preferably from 100 to 500 mmol/L.

### Industrial Applicability

A method of stabilizing an enzyme and an enzyme composition of the present invention are concerned with a control substance used in medical examination and are capable of sufficiently stabilizing AST contained in serum, a buffer or a medium such as soluble protein solution. Thus, the present :invention has industrial applicability by providing a method of and a material for obtaining accurate and stable examined data in the field of the medical clinical examination.

## Claims

1. A method for stabilizing an aspartate aminotransferase in at least one medium selected from the group consisting of serum and buffer, the method comprising adding proline to said medium.

2. The method according to claim 1, wherein the concentration of said proline is within a range from 0.5 to 500 mmol/L .

3. The method according to claim 1, wherein said buffer is a buffer containing a soluble protein.

4. The method according to claim 3, wherein said soluble protein is at least one soluble protein selected from the group consisting of albumin and gelatin.

5. The method according to claim 4, wherein the concentration of said albumin is within a range from 0.5 to 15 W/V%.

6. The method according to claim 4, wherein the concentration of said gelatin is within a range from 0.5 to 15 W/V%.

7. An enzyme composition comprising at least one medium selected from the group consisting of serum and buffer, an aspartate aminotransferase and proline.

8. The enzyme composition according to claim 7, wherein the concentration of said proline is within a range from 0.5 to 500 mmol/L.

9. The enzyme composition according to claim 7, wherein the said buffer is a buffer containing a soluble protein.

10. The enzyme composition according to claim 9, wherein said soluble protein is at least one soluble protein selected from the group consisting of albumin and gelatin.

11. The enzyme composition according to claim 10, wherein the concentration of said albumin is within a range from 0.5 to 15 W/V%.

12. The enzyme composition according to claim 10, wherein the concentration of said gelatin is within a range from 0.5 to 15 W/V%.

## Patentansprüche

1. Verfahren zum Stabilisieren einer Aspartat-Aminotransferase in mindestens einem Medium, welches aus der Gruppe gewählt ist, die aus Serum und Puffer besteht, wobei das Verfahren ein Hinzufügen von Prolin zu dem Medium umfasst.

2. Verfahren gemäß Anspruch 1, wobei sich die Konzentration des Prolins innerhalb eines Bereichs von 0,5 bis 500 mMol/l befindet.

3. Verfahren gemäß Anspruch 1, wobei der Puffer ein Puffer ist, welcher ein lösliches Protein beinhaltet.

4. Verfahren gemäß Anspruch 3, wobei das lösliche Protein mindestens ein lösliches Protein ist, welches aus der Gruppe gewählt ist, die aus Albumin und Gelatine besteht.

5. Verfahren gemäß Anspruch 4, wobei sich die Konzentration des Albumins innerhalb eines Bereichs von 0,5 bis 15 W/V% befindet.

6. Verfahren gemäß Anspruch 4, wobei sich die Konzentration der Gelatine innerhalb eines Bereichs von 0,5 bis 15 W/V% befindet.

7. Enzym-Zusammensetzung, umfassend mindestens ein Medium, welches aus der Gruppe gewählt ist, die aus Serum und Puffer, einer Aspartat-Aminotransferase und Prolin besteht.

8. Enzym-Zusammensetzung gemäß Anspruch 7, wobei sich die Konzentration des Prolins innerhalb eines Bereichs von 0,5 bis 500 mmol/l befindet.

9. Enzym-Zusammensetzung gemäß Anspruch 7, wobei der Puffer ein Puffer ist, der ein lösliches Protein enthält.

10. Enzym-Zusammensetzung gemäß Anspruch 9, wobei das lösliche Protein mindestens ein lösliches Protein ist, welches aus der Gruppe gewählt ist, die aus Albumin und Gelatine besteht.

11. Enzym-Zusammensetzung gemäß Anspruch 10, wobei sich die Konzentration des Albumins innerhalb eines Bereichs von 0,5 bis 15 W/V% befindet.

12. Enzym-Zusammensetzung gemäß Anspruch 10, wobei sich die Konzentration der Gelatine innerhalb eines Bereichs von 0,5 bis 15 W/V% befindet.

## Revendications

1. Procédé de stabilisation d'une aspartate aminotransférase dans au moins un milieu choisi dans le groupe constitué par un sérum et un tampon, le procédé comprenant l'addition de proline audit milieu.

2. Procédé selon la revendication 1, dans lequel la concentration de ladite proline se situe dans une plage de 0,5 à 500 mmol/l.

3. Procédé selon la revendication 1, dans lequel ledit tampon est un tampon contenant une protéine soluble.

4. Procédé selon la revendication 3, dans lequel ladite protéine soluble est au moins une protéine soluble choisie dans le groupe constitué par l'albumine et la gélatine.

5. Procédé selon la revendication 4, dans lequel la concentration de ladite albumine se situe dans une plage de 0,5 à 15 % p/v.

6. Procédé selon la revendication 4, dans lequel la concentration de ladite gélatine se situe dans une plage de 0,5 à 15 % p/v.

7. Composition d'enzyme comprenant au moins un milieu choisi dans le groupe constitué par un sérum et un tampon, une aspartate aminotransférase et de la proline.

8. Composition enzymatique selon la revendication 7, dans laquelle la concentration de ladite proline se situe dans une plage de 0,5 à 500 mmol/l.

9. Composition enzymatique selon la revendication 7, dans laquelle ledit tampon est un tampon contenant une protéine soluble.

10. Composition enzymatique selon la revendication 9, dans laquelle ladite protéine soluble est au moins une protéine soluble choisi dans le groupe constitué par l'albumine et la gélatine.

11. Composition enzymatique selon la revendication 10, dans laquelle la concentration de ladite albumine se situe dans une plage de 0,5 à 15 % p/v.

12. Composition enzymatique selon la revendication 10, dans laquelle la concentration de ladite gélatine se situe dans une plage de 0,5 à 15 % p/v.
